(19) Europäisches Patentamt
European Patent Office
Office européen des brevets

(11) **EP 4 781 915 A1**

(12) **EUROPEAN PATENT APPLICATION**
published in accordance with Art. 153(4) EPC

(43) Date of publication:
29.07.2026 Bulletin 2026/31

(21) Application number: 25804568.1

(22) Date of filing: 15.09.2025

(51) International Patent Classification (IPC):
*A61B 8/00* (2006.01)

(52) Cooperative Patent Classification (CPC):
A61B 8/00; A61B 8/08; A61M 35/00; B25J 11/00;
B25J 18/00

(86) International application number:
PCT/KR2025/014323

(87) International publication number:
WO 2026/054626 (12.03.2026 Gazette 2026/11)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH LA MA MD TN

(30) Priority: 06.09.2024 KR 20240121337

(71) Applicant: Medicalpark Co., Ltd.
Yongin-si, Gyeonggi-do 16827 (KR)

(72) Inventor: PARK, Hee Boong
Seoul 06281 (KR)

(74) Representative: BCKIP Part mbB
MK1
Landsbergerstraße 98, 3.Stock
80339 München (DE)

Remarks:
The filing date of the international application is
within two months from the date of expiration of the
priority period (R. 26bis.3 PCT)

(54) **ROBOT ARM-ASSISTED ULTRASONIC MEDICAL IMAGING SYSTEM AND ROBOT ARM-ASSISTED ULTRASONIC MEDICAL IMAGE ACQUISITION METHOD**

(57) A medical imaging system according to the present invention includes a multi-axis robotic arm, a multi-axis robotic arm control device that controls the multi-axis robotic arm, an ultrasonic probe mounted on the multi-axis robotic arm, an ultrasonic image generation device that controls the ultrasonic probe to generate an ultrasonic image, and a three-dimensional (3D) image capturing device. Further, the medical imaging system includes an ultrasound gel dispenser that is mounted on the multi-axis robotic arm and in which ultrasound gel is accommodated, a nozzle disposed adjacent to the ultrasonic probe to receive the ultrasound gel from the ultrasound gel dispenser and discharge the received ultrasound gel forward in a movement direction of the ultrasonic probe, and a dispenser control device that controls discharge of the ultrasound gel accommodated in the ultrasound gel dispenser. Further, the medical imaging system includes a computer that controls the multi-axis robotic arm control device, the ultrasonic image generation device, the 3D image capturing device, and the dispenser control device.

FIG. 3

# Description

[Technical Field]

The present invention relates to a robot-arm assisted ultrasound medical imaging system, and more specifically, to an ultrasound medical imaging system for applying ultrasound gel using a multi-axis robotic arm and simultaneously acquiring an ultrasonic image.

Further, the present invention relates to a robot-arm assisted ultrasound medical image acquisition method, and more specifically, to a method of acquiring an ultrasound medical image while applying ultrasound gel along a predetermined path using a robotic arm.

[Background Art]

Ultrasound examination devices are generally used to examine diseases of human body parts such as thyroid gland, musculoskeletal system, breast, etc. An ultrasonic image (or ultrasonography) is an image formed by radiating pulsed waves into human tissues with different acoustic impedances, receiving reflected signals, and amplifying and converting the received signals.

When air is present between an ultrasonic (or ultrasound) probe and a skin surface while an ultrasound examination is performed, it is difficult to acquire high-quality ultrasonic images because the incident ultrasound waves are reflected by the air. Therefore, ultrasound gel is applied on the skin surface in order to prevent ultrasound reflection caused by air present between the ultrasonic probe and the skin surface. Further, the ultrasound gel applied on the skin surface incidentally helps the ultrasonic probe to move smoothly while the ultrasonic probe is in contact with the skin surface. In ultrasound examinations, an operator generally applies ultrasound gel directly on a skin surface of a human body using his/her hands or an instrument.

Meanwhile, when an operator holds an ultrasonic probe and conducts a diagnosis for a long period of time, it can cause pain due to strain on his/her shoulders and arms, resulting in a loss of diagnostic focus on an ultrasonic image. In order to address these problems, ultrasound systems that can acquire ultrasonic images using an ultrasonic probe mounted on a robotic arm are being developed.

For example, in US Laid-Open Patent Publication No. US2012/0271173 A1 (titled AUTOMATIC ULTRASONIC SCANNING SYSTEM AND SCANNING METHOD THEREOF), a system for acquiring an ultrasonic image using a robotic arm is disclosed. The system disclosed in the patent document includes a multi-axis robotic arm, an ultrasonic probe, a three-dimensional (3D) image capture device, and a computer. The 3D image capture device includes a depth camera installed to photograph an ultrasound scan area of a patient. The computer processes an image captured by the depth camera to generate a scan path for acquiring an ultrasonic image of the patient by moving the ultrasonic probe mounted on the robotic arm and controls the multi-axis robotic arm to move the ultrasonic probe and acquire an ultrasonic image of the patient. In the system disclosed in the patent document, the idea of automatically applying ultrasound gel on the patient's skin surface in the case of acquiring the ultrasonic image using the robotic arm is not disclosed.

Further, in US Laid-Open Patent Publication No. US2008/0021317 A1 (title of the invention, ULTRASOUND MEDICAL IMAGING WITH ROBOTIC ASSISTANCE FOR VOLUME MAGING), a system for acquiring an ultrasonic image using a robotic arm is disclosed. The system disclosed in the patent document includes a robot mechanism, an ultrasonic probe (transducer) mounted on the robot mechanism, a force sensor, an ultrasound imaging system, a processor, and a digitizer. The digitizer may acquire 3D surface information of a body part to be scanned with the ultrasonic probe, and the processor may determine information on a position to be scanned with the ultrasonic probe using the 3D surface information and control the robot mechanism to automatically acquire an ultrasonic image. Further, in identification number [0038] of the patent document, the robot mechanism is described as including a gel dispenser and a suction spout and being configured to supply gel from a gel storage to the robot mechanism through a tube. Further, the gel dispenser is described as being positioned adjacent to the ultrasonic probe (transducer) and supplying gel from the gel dispenser to the patient using a pump.

[Description of Invention]

[Technical Problem]

However, in the above-described patent documents, a specific technical configuration of the gel dispenser is not disclosed. Further, the system disclosed in the patent document is equipped with a force sensor or a pressure sensor and is configured to measure force with which the ultrasonic probe comes into contact with the patient when an ultrasonic image is acquired using a robot mechanism. That is, the system disclosed in the patent document is formed so that, in the case of acquiring the ultrasonic image, the ultrasonic probe mounted on the robot mechanism comes into contact with the patient and pressurizes the patient with a certain pressure, and the system uses the force sensor to prevent excessive force from being applied to the patient.

In the case of acquiring an ultrasonic image by scanning the patient's skin surface using the ultrasonic probe mounted on the robotic arm, it is necessary to prevent ultrasound reflection caused by air to acquire a high-quality ultrasonic image. To this end, a device capable of applying ultrasound gel on the patient's skin surface at the same time that the ultrasonic probe mounted on the robotic arm moves is required. Further, when applying the ultrasound gel on the skin surface, a device capable of automatically applying ultrasound gel that is designed to prevent air bubbles from being mixed with the ultrasound gel is required.

Further, there is a need for an ultrasonic image acquisition system using a robotic arm that can acquire an ultrasonic image without directly applying force to a patient by an ultrasonic probe when the ultrasonic probe mounted on the robotic arm moves along a scan path. That is, there is a need for a system capable of acquiring an ultrasonic image by moving an ultrasonic probe mounted on a robotic arm along a scan path so that the ultrasonic probe does not come into direct contact with a patient's skin but comes into contact with ultrasound gel applied on a skin surface and simultaneously moving the ultrasonic probe along the patient's skin surface.

Meanwhile, providing an ultrasonic image of an entire cross section of a portion from which an ultrasonic image is to be acquired, such as the patient's breast area, may be more useful in diagnosing the patient's disease. However, a linear array ultrasonic probe has a length of about 4 to 5 cm and cannot acquire an ultrasound image of the entire breast area in a single ultrasound scan. The ultrasonic image of the entire cross section of the breast area may be acquired by synthesizing a plurality of cross-sectional images with respect to the same surface. There is a need for a system capable of acquiring a plurality of cross-sectional ultrasound images at a desired position using a multi-axis robotic arm and synthesizing the plurality of cross-sectional ultrasound images to provide an extended ultrasonic image.

The present invention is intended to solve the above problems required when the ultrasonic image of the patient is acquired using the multi-axis robotic arm.

The present invention is directed to providing a new ultrasound medical imaging system capable of automatically supplying ultrasound gel when an ultrasonic probe mounted on a robotic arm moves.

Further, the present invention is directed to providing a new ultrasound medical image the device configured to avoid applying excessive force to a patient when an ultrasonic probe mounted on a robotic arm moves.

Further, the present invention is directed to providing a method of acquiring an ultrasound medical image using a new robot-arm assisted ultrasound medical imaging system.

Further, the present invention is directed to providing a method of forming an extended ultrasonic image using a new robot-arm assisted ultrasound medical imaging system.

[Technical Solution]

According to an aspect of the present invention, a robot-arm assisted ultrasound medical imaging system is provided. The medical imaging system according to the present invention includes a multi-axis robotic arm, a multi-axis robotic arm control device that controls the multi-axis robotic arm, an ultrasonic probe mounted on the multi-axis robotic arm, an ultrasonic image generation device that controls the ultrasonic probe to generate an ultrasonic image, and a three-dimensional (3D) image capturing device. Further, the medical imaging system includes an ultrasound gel dispenser that is mounted on the multi-axis robotic arm and in which ultrasound gel is accommodated, a nozzle disposed adjacent to the ultrasonic probe so as to receive the ultrasound gel from the ultrasound gel dispenser and discharge the received ultrasound gel forward in a movement direction of the ultrasonic probe, and a dispenser control device that controls discharge of the ultrasound gel accommodated in the ultrasound gel dispenser. Further, the medical imaging system includes a computer that controls the multi-axis robotic arm control device, the ultrasonic image generation device, the 3D image capturing device, and the dispenser control device.

In particular, in the medical imaging system according to the present invention, the computer may be configured to receive an image of an ultrasound scan area of a patient from the 3D image capturing device, process the image of the ultrasound scan area of the patient to generate 3D shape data of the ultrasound scan area, generate an ultrasound scan path for acquiring the ultrasonic image by moving the ultrasonic probe mounted on the multi-axis robotic arm on the basis of the 3D shape data of the ultrasound scan area, and control the multi-axis robotic arm control device, the ultrasonic image generation device, and the dispenser control device to discharge the ultrasound gel and simultaneously acquire the ultrasonic image while the ultrasonic probe moves along the ultrasound scan path.

In some embodiments, the ultrasound scan path generated by the computer may be formed such that an end portion of the ultrasonic probe is spaced a certain distance from a surface of an ultrasound scan area of the patient.

In some embodiments, the ultrasound gel dispenser may include a hollow housing having an inlet formed at one side thereof and connected to an air source to allow high-pressure air to be introduced and an opening formed at the other side to allow a flexible ultrasound gel container to be inserted therein, and mounted on the multi-axis robotic arm, and a hollow housing cap having a small diameter portion formed to allow an outlet of the flexible ultrasound gel container inserted therein to be inserted and a large diameter portion formed to be sealed and coupled to the opening of the hollow housing. Further, the dispenser control device may include a valve for allowing or blocking a flow of air of the air source supplied to the hollow housing of the ultrasound gel dispenser.

In some embodiments, the ultrasonic probe may be a linear array probe, the nozzle may include an inlet, an outlet, and a passage that connects the inlet and the outlet, and the passage may be formed such that a length of a cross section thereof increases from the inlet to the outlet and is greater than a length of the linear array probe.

In some embodiments, an end portion of the nozzle may be disposed to be spaced a certain distance from the end portion of the ultrasonic probe so as to be positioned further from the patient's skin surface than the end portion of the ultrasonic probe.

In some embodiments, the nozzle may be formed to surround a side surface in a width direction of the linear array probe so that the side surface in the width direction of the linear array probe defines the passage.

According to another aspect of the present invention, a robot-arm assisted ultrasonic image acquisition method is provided. The robot-arm assisted ultrasound medical image acquisition method according to the present invention is a method of acquiring an ultrasound medical image using the robot-arm assisted ultrasound medical imaging system.

The robot-arm assisted ultrasound medical image acquisition method according to the present invention includes receiving, by a computer, an image of an ultrasound scan area of a patient from a depth imaging device, processing, by the computer, the received image of the ultrasound scan area of the patient and generating 3D shape data of the ultrasound scan area, generating, by the computer, an ultrasound scan path for acquiring an ultrasonic image by moving an ultrasonic probe mounted on a multi-axis robotic arm on the basis of the generated 3D shape data of the ultrasound scan area, and controlling, by the computer, the multi-axis robotic arm, the ultrasonic probe mounted on the multi-axis robotic arm, and an ultrasound gel supply device mounted adjacent to the ultrasonic probe, and discharging ultrasound gel forward in a movement direction of the ultrasonic probe and simultaneously acquiring the ultrasonic image while the ultrasonic probe moves along the ultrasound scan path.

In some embodiments, the ultrasound scan path generated by the computer may be formed such that an end portion of the ultrasonic probe is spaced a certain distance from a surface of the ultrasound scan area of the patient. Further, the ultrasound gel supply device may include an ultrasound gel dispenser that is mounted on the multi-axis robotic arm and in which ultrasound gel is accommodated, a nozzle disposed adjacent to the ultrasonic probe to receive the ultrasound gel from the ultrasound gel dispenser and discharge the received ultrasound gel forward in the movement direction of the ultrasonic probe, and a dispenser control device that controls discharge of the ultrasound gel accommodated in the ultrasound gel dispenser, and the computer may be configured to control the dispenser control device to discharge the ultrasound gel.

In some embodiments, the ultrasonic probe may be a linear array probe, and the computer may be configured to control the ultrasound gel dispenser to discharge the ultrasound gel so that a certain distance (G1) between an end portion of the linear array probe and the surface of the ultrasound scan area of the patient is greater than a product of a length of the linear array probe and a moving speed of the linear array probe.

According to still another aspect of the present invention, a method of acquiring an extended ultrasonic image is provided.

The method of acquiring an extended ultrasonic image according to the present invention is a method of acquiring an extended ultrasonic image using a multi-axis robotic arm equipped with an ultrasonic probe, a computer, and a 3D image capturing device. The method of acquiring an extended ultrasonic image according to the present invention includes photographing, by the 3D image capturing device, an ultrasound scan area of a patient, receiving, by the computer, an image of the ultrasound scan area from the 3D image capturing device and generating 3D shape data, generating, by the computer, an extended image plane for acquiring an extended ultrasonic image for the generated 3D shape data, generating, by the computer, a line scan path for acquiring a plurality of ultrasonic image frames including an overlapping area with respect to the extended image plane, controlling, by the computer, a robotic arm equipped with the ultrasonic probe to move the ultrasonic probe along the generated line scan path and acquire a plurality of ultrasonic image frames, selecting, by the computer, ultrasonic image frames corresponding to the extended image plane from among the plurality of acquired ultrasonic image frames, and generating, by the computer, an extended ultrasonic image by synthesizing overlapping portions of the plurality of selected ultrasonic image frames.

In some embodiments, the acquiring of the plurality of ultrasonic image frames may further include controlling, by the computer, the multi-axis robotic arm, the ultrasonic probe mounted on the multi-axis robotic arm, and the ultrasound gel supply device, and discharging ultrasound gel forward in a movement direction of the ultrasonic probe and simultaneously acquiring an ultrasonic image while the ultrasonic probe moves along the line scan path.

In some embodiments, the extended image plane generated in the generating of the extended image plane may be a plane parallel to a Y-Z plane with respect to reference coordinates of the multi-axis robot arm.

In some embodiments, the line scan path generated by the computer may be formed such that an end portion of the ultrasonic probe is spaced a certain distance (G1) from a surface of the ultrasound scan area of the patient.

[Advantageous Effects]

A robot-arm assisted ultrasound medical imaging system according to the present invention is configured to apply ultrasound gel on a patient's skin surface at the same time that an ultrasonic probe mounted on a robotic arm moves, and thus it is possible to prevent ultrasound reflection caused by air on the skin surface to acquire ahigh-quality ultrasonic image.

Further, in the robot-arm assisted ultrasound medical imaging system according to the present invention, it is possible to prevent an ultrasound probe from applying excessive force to a patient by moving the ultrasonic probe mounted on the robotic arm along a scan path so that the ultrasonic probe does not come into direct contact with the patient's skin but comes into contact with the ultrasound gel applied on the skin surface and simultaneously moving the ultrasonic probe along the patient's skin surface.

[Description of Drawings]

FIG. 1 is a block diagram illustrating a multi-axis robot-arm assisted ultrasound medical imaging system according to the present invention.

FIG. 2 is a perspective view of a robot-arm assisted ultrasound medical imaging system according to an embodiment of the present invention.

FIG. 3 is a side view illustrating the installation state of a robotic arm, an ultrasound gel dispenser, and an ultrasonic probe in the embodiment illustrated in FIG. 2.

FIG. 4 is a perspective view of a hand, an ultrasonic probe, a nozzle, and a three-way solenoid valve that are mounted on a robotic arm according to an embodiment.

FIG. 5 is a perspective view of a nozzle that surrounds the ultrasonic probe illustrated in FIG. 4.

FIG. 6 is a plan view of the nozzle illustrated in FIG. 5.

FIG. 7 is a cross-sectional view along line X-X of FIG. 6.

FIG. 8 is an explanatory diagram of a passage, which is formed by a side surface of the ultrasonic probe, of the nozzle illustrated in FIG. 5.

FIG. 9 is a cross-sectional view of an ultrasound gel dispenser according to an embodiment of the present invention.

FIG. 10 is an explanatory diagram illustrating a state in which the ultrasound gel dispenser illustrated in FIG. 9 is separated.

FIG. 11 is an explanatory diagram illustrating a state in which ultrasound gel is discharged from the ultrasound gel dispenser illustrated in FIG. 9.

FIG. 12 is an explanatory diagram of a path along which ultrasound scanning is performed on an ultrasound area of a patient's thyroid gland.

FIG. 13 is an explanatory diagram of a path along which ultrasound scanning is performed on an ultrasound area of a patient's musculoskeletal system.

FIGS. 14 and 15 are explanatory diagrams illustrating states in which ultrasound gel is supplied from a nozzle when line scanning is performed on a skin surface with an ultrasonic probe.

FIG. 16 is a flowchart illustrating a multi-axis robot-arm assisted ultrasound medical image acquisition method according to the present invention.

FIG. 17 is an explanatory diagram of a path along which ultrasound scanning is performed on an ultrasound area of a patient's right breast.

FIG. 18 is an explanatory diagram illustrating a state in which an ultrasound area of a patient's right breast is scanned in an overlapping manner.

FIG. 19 is an explanatory diagram illustrating an extended image plane.

FIG. 20 is a flowchart illustrating a method of forming an extended ultrasonic image according to the present invention.

FIG. 21 illustrates embodiments of image frames corresponding to an extended image plane I4 among a plurality of image frames on an ultrasound scan path.

FIG. 22 illustrates an extended ultrasonic image acquired by overlapping and synthesizing the image frames illustrated in FIG. 21 according to an embodiment.

[Modes of the Invention]

Other objects, specific advantages, and new features of the present invention will be clearly understood through the following detailed description and exemplary embodiments taken in conjunction with the accompanying drawings. In describing the present invention, the size and shape of components illustrated in the drawings may be exaggerated or simplified for clarity and convenience of description. Further, terms specifically defined in consideration of the configuration and operation of the present invention may vary depending on a user or operator's intentions or customs. These terms should be interpreted as meanings and concepts consistent with the purpose and effect of the present invention based on the contents throughout this specification.

Hereinafter, exemplary embodiments of the present invention will be described in detail with reference to the accompanying drawings.

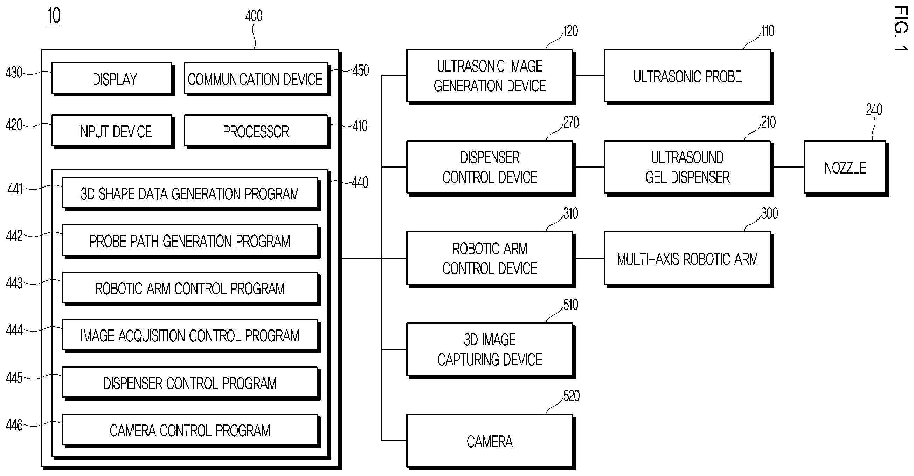

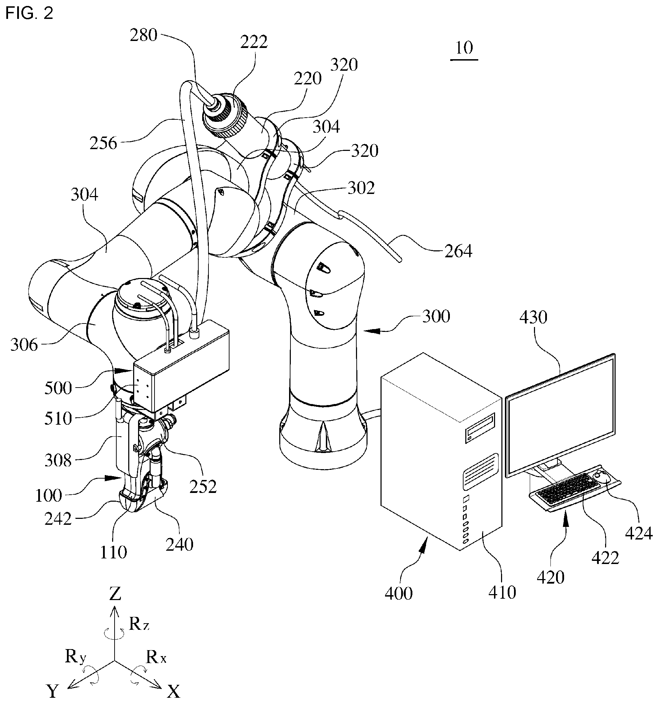

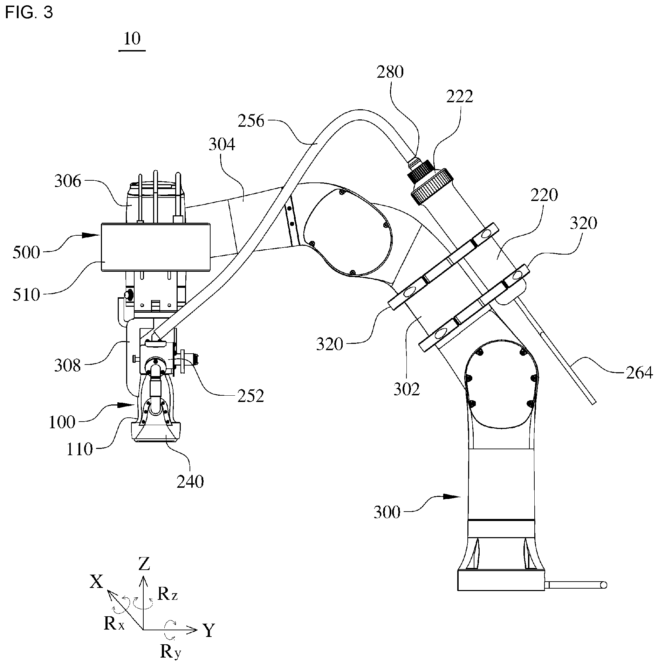

FIG. 1 is a block diagram illustrating a multi-axis robot-arm assisted ultrasound medical imaging system according to the present invention, and FIG. 2 is a perspective view of a robot-arm assisted ultrasound medical imaging system according to an embodiment of the present invention. FIG. 3 is a side view illustrating the installation state of a robotic arm, an ultrasound gel dispenser, and an ultrasonic probe in the embodiment illustrated in FIG. 2.

Referring to FIGS. 1 to 3, a medical imaging system 10 according to the present invention includes a multi-axis robotic arm 300, a multi-axis robotic arm control device 310 for controlling the multi-axis robotic arm 300, an ultrasonic image acquisition device 100, and a three-dimensional (3D) image capturing device 510. Further, a separate camera 520 for capturing a two-dimensional (2D) image may be further provided.

The ultrasonic image acquisition device 100 includes an ultrasonic probe 110 mounted on the multi-axis robotic arm 300 and an ultrasonic image generation device 120 for controlling the ultrasonic probe 110 to generate an ultrasonic image. The ultrasonic probe 110 transmits an ultrasound signal to a patient and receives an ultrasound reflection signal (echo) from the inside of the patient's body. Various ultrasonic probes 110 are known. For example, a line array probe, a phase array probe, a convex probe, etc. are known, and an appropriate ultrasonic probe may be selected and used according to the needs of the system of the present invention. The ultrasonic image generation device 120 includes a processor and may control the ultrasonic probe 110 to acquire an ultrasonic image and transmit the acquired ultrasonic image to an external device.

The multi-axis robotic arm 300 is a robotic arm capable of motion control in at least 5 axes, preferably 6 axes. The multi-axis robotic arm 300 may have a plurality of joint arms 302, 304, and 306 and a hand 308, and may be a four-axis robotic arm or vertical multiple joint robotic arm capable of performing X-Y-Z axis translational movements and a Z-axis rotational movement $R_z$. Further, the multi-axis robotic arm 300 may be a six-axis multiple joint robotic arm capable of X-Y-Z axis translational movements and X-Y-Z axis rotational movements $R_x$, $R_y$, and $R_z$. The multi-axis robotic arm control device 310 moves the ultrasonic probe 110 to a determined position and controls a pose for acquiring an ultrasonic image according to a robotic arm control program 443 of a computer 400. That is, the robotic arm 300 may place the ultrasonic probe 110 at an arbitrary position and in an arbitrary pose in a 3D space and move the ultrasonic probe 110 at an arbitrary trajectory and at an arbitrary speed.

The 3D image capturing device 510 is a device that photographs the patient's ultrasonic image scan area and provides 3D shape information. A depth camera or a 3D camera may be used as the 3D image capturing device 510. The depth camera provides depth information of the captured image. The 3D camera 510 may be mounted on the joint arm 306 equipped with the ultrasonic probe 110 of the multi-axis robotic arm 300, as illustrated in FIGS. 2 and 3. Further, the 3D camera 510 and the 2D camera 520 may be mounted independently of the multi-axis robotic arm 300 on a stand, a wall, or the like.

Further, the medical imaging system 10 according to the present invention includes an ultrasound gel supply device 200 for applying ultrasound gel on the patient's ultrasound scan area. The ultrasound gel supply device 200 is a device for continuously supplying ultrasound gel onto the patient's skin surface when the ultrasonic probe 110 mounted on the multi-axis robotic arm 300 moves. The ultrasound gel supply device 200 includes an ultrasound gel dispenser 210, a nozzle 240, and a dispenser control device 270. The ultrasound gel dispenser 210 is mounted on the multi-axis robotic arm 300 and contains ultrasound gel. The nozzle 240 is mounted adjacent to the ultrasonic probe 110 to receive the ultrasound gel from the ultrasound gel dispenser 210 and discharge the ultrasound gel forward in a movement direction of the ultrasonic probe 110. The dispenser control device 270 is a device for controlling the discharge of the ultrasound gel accommodated in the ultrasound gel dispenser 210.

Referring to FIGS. 2 and 3, the ultrasound gel dispenser 210 is clamped to be detachable from the joint arm 302 by a plurality of clamps 320. Further, the ultrasonic probe 110 is fixed to the hand 308 of the robotic arm 300, and a pair of nozzles 240 and 242 are fixed to the ultrasonic probe 110. Further, a three-way solenoid valve 252 for selectively supplying ultrasound gel to the pair of nozzles 240 and 242 are fixed to the hand 308. The ultrasound gel dispenser 210 and the three-way solenoid valve 252 are connected by a flexible hose 256 through which the ultrasound gel passes. Instead of the three-way solenoid valve 252 that can automatically change a flow direction of the ultrasound gel, a three-way manual valve that can selectively control the supply of ultrasound gel 30 by manual operation of a handle may be used. Further, a relief valve (lift valve) may be connected to the flexible hose 256. When pressure greater than or equal to a determined pressure is applied, the relief valve opens and allows the ultrasound gel in the flexible hose 256 to be discharged to the outside, thereby protecting the device. Further, a check valve may be installed in the hose 256 to prevent a backflow of the ultrasound gel.

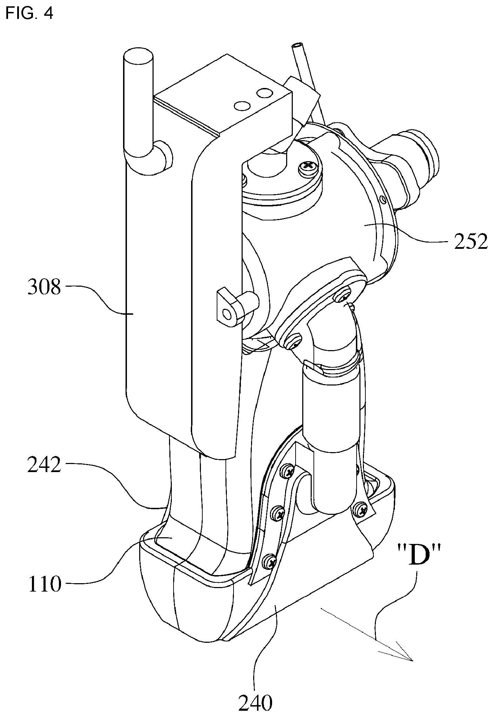

FIG. 4 is a perspective view of a hand 308, an ultrasonic probe 110, a nozzle 240, and a three-way solenoid valve 252 that are mounted on a robotic arm according to an embodiment. In the present embodiment, the ultrasonic probe 110 may be a linear array probe. The nozzle 240 is disposed in front of the ultrasonic probe 110 in a movement direction (arrow direction D). As illustrated above, the nozzle 240 is formed to surround an outer surface of the ultrasonic probe 110.

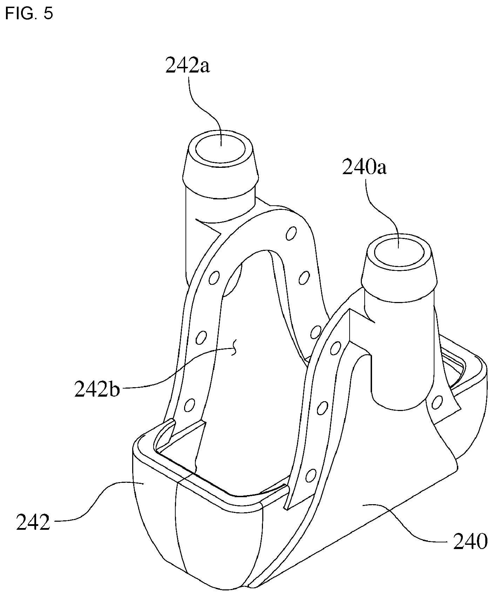

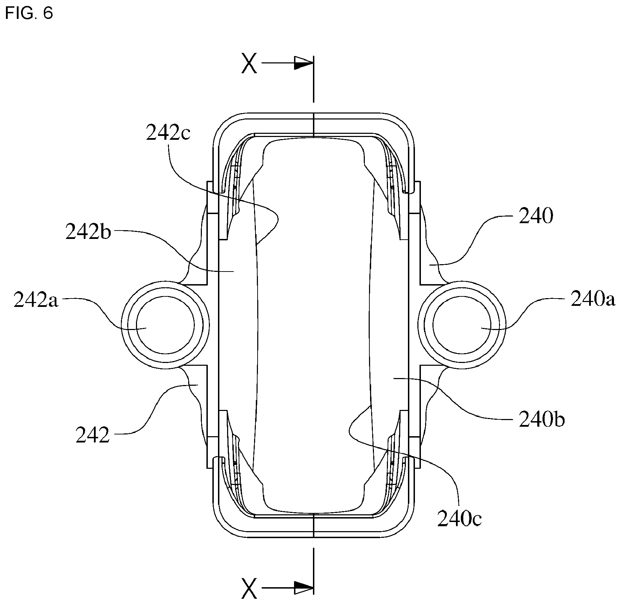

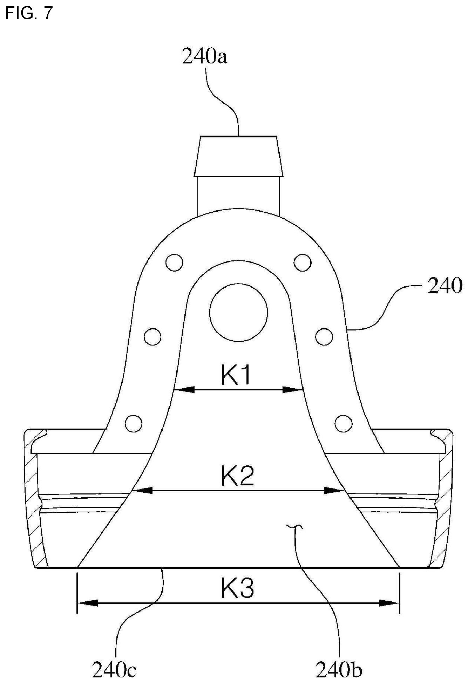

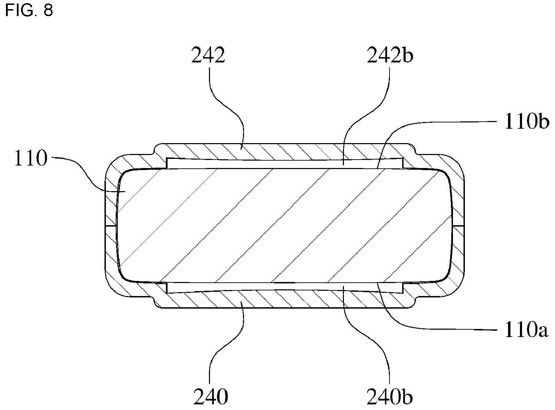

FIG. 5 is a perspective view of a nozzle that surrounds the ultrasonic probe illustrated in FIG. 4, FIG. 6 is a plan view of the nozzle illustrated in FIG. 5, and FIG. 7 is a cross-sectional view along line X-X of FIG. 6. Further, FIG. 8 is an explanatory diagram of a passage, which is formed by a side surface of the ultrasonic probe, of the nozzle illustrated in FIG. 5.

Referring to FIGS. 5 to 7, the nozzle 240 includes an inlet 240a through which the ultrasound gel is supplied, an outlet 240c through which the ultrasound gel is discharged, and a passage 240b that connects the inlet 240a and the outlet 240c. Referring to FIG. 8, in the present embodiment, the passage 240b and outlet 240c of the nozzle 240 are formed to be defined by one side surface 110a in a width direction of the ultrasonic probe 110 that is surrounded by the nozzle 240. In some embodiments, unlike the nozzle of the present embodiment, the passage and the outlet may be formed not by using the one side surface 110a in the width direction of the ultrasonic probe 110, but by forming a separate wall corresponding to a side surface of the ultrasonic probe.

Referring to FIG. 7, the passage 240b of the nozzle 240 of the present embodiment is formed such that a length of a cross section thereof increases from the inlet 240a to the outlet 240c, that is, the length relationship is K3>K2>K1. In some embodiments, it is preferable that the length K3 of the cross section of the outlet 240c be greater than a length of an array direction of the ultrasonic probe 110. Each of the nozzles 240 and 242 allows the ultrasound gel to be widely spread and discharged onto the patient's skin through the passage 248 having an inverted funnel shape.

Referring to FIG. 4, the nozzle 242 is formed to surround the other side surface 110b in the width direction of the ultrasonic probe 110 and is disposed in an opposite direction to a movement direction of the ultrasonic probe 110 as illustrated. Therefore, the three-way solenoid valve 252 allows the ultrasound gel to flow through the nozzle 240 disposed in the movement direction of the ultrasonic probe 110 and blocks the flow of the ultrasound gel to the nozzle 242.

In some embodiments, only one nozzle 240 may be used to be mounted on the one side surface 110a in the width direction of the ultrasonic probe 110. In this case, the hose 256 may be directly connected to the nozzle 240 without using the three-way solenoid valve 252 for selectively supplying the ultrasound gel to the nozzle.

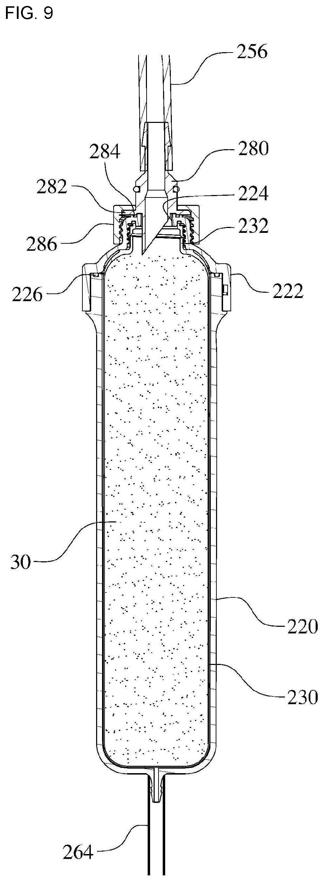

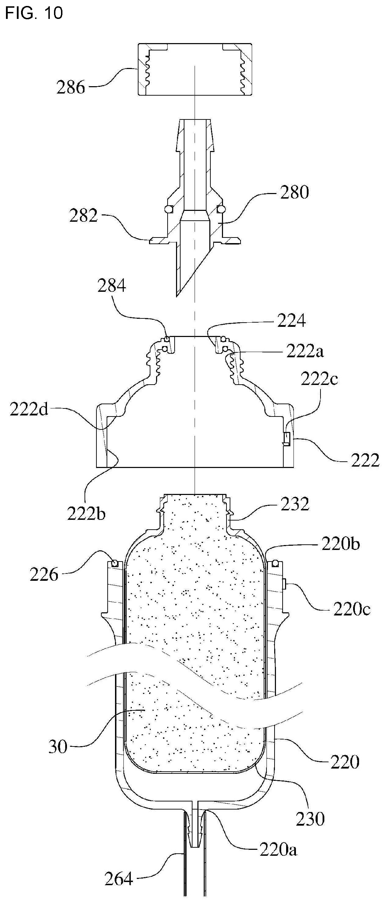

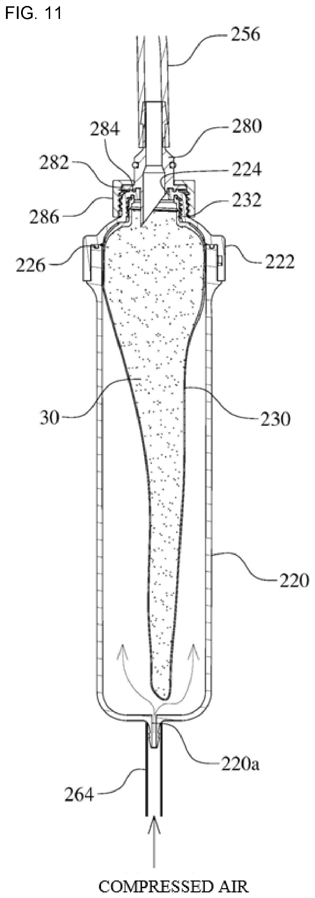

FIG. 9 is a cross-sectional view of an ultrasound gel dispenser according to an embodiment of the present invention, FIG. 10 is an explanatory diagram illustrating a state in which the ultrasound gel dispenser illustrated in FIG. 9 is separated, and FIG. 11 is an explanatory diagram illustrating a state in which ultrasound gel is discharged from the ultrasound gel dispenser illustrated in FIG. 9.

Referring to FIGS. 9 and 10, an ultrasound gel dispenser 210 includes a hollow housing 220 and a housing cap 222. At one side of the hollow housing 220, an inlet 220a that is connected to an air source and allows high-pressure air to be introduced therein is formed, and at the other side of the hollow housing 220, an opening 220b that allows a flexible ultrasound gel container 230 to be inserted into a hollow interior is provided. The hollow housing 220 is mounted on the joint arm 302 by a plurality of clamps 320, as illustrated in FIG. 3. The ultrasound gel container 230 is a flexible container that may be flexibly deformed when an external force is applied, such as a container containing toothpaste, and a screw for screw fastening to a screw formed on an inner circumferential surface of the housing cap 222 is formed at the outlet 232. The hollow housing cap 222 includes a small diameter portion 222a formed to allow the outlet 232 of the flexible ultrasound gel container 230 inserted therein to be inserted, and a large diameter portion 222b formed to be sealed and coupled to the opening 220b of the hollow housing 220. A spiral groove 222c may be formed in an inner circumferential surface of the large diameter portion 222b in order to be sealed and coupled to the opening 220b of the hollow housing 220 and a spiral protrusion 220c may be formed on an outer circumferential surface of the opening 220b, or a screw thread for screw-fastening the inner circumferential surface of the large diameter portion 222b to the outer circumferential surface of the opening 220b may be formed. Further, an O-ring 226 may be mounted at an end portion of the opening 220b, and a step 222d for pressing the O-ring 226 to the large diameter portion 222b may be formed.

The ultrasound gel dispenser 210 may further include a tube coupling 280 or tube fitting for connecting one end of the flexible hose 256 to the outlet 232 of the ultrasound gel container 230. The tube coupling 280 is inserted into the outlet 232 of the ultrasound gel container 230 through a hole 224 formed in the small diameter portion 222a of the housing cap 222. A flange 282 is formed on an outer circumferential surface of the tube coupling 280, and a plurality of O-rings 284 are installed between the housing cap 222 and the tube coupling 280 and between the housing cap 222 and the ultrasound gel container 230 for sealing. Further, an open cap 286 is detachably screw-fastened to an outer circumferential surface of the small diameter portion 222a of the housing cap 222 for fixing the tube coupling 280. The flange 282 is engaged with the inner surface of the open cap 286 to prevent the tube coupling 280 from being separated from the gel container 230. A flexible hose 264 for supplying high-pressure air is connected to the inlet 220a of the hollow housing 220. Although not illustrated, the flexible hose 264 is connected to a high-pressure air tank storing high-pressure air or to an air compressor for supplying high-pressure air.

The dispenser control device 270 may include a microprocessor. Further, the dispenser control device 270 may include a flow rate control valve for controlling a flow rate at which air is supplied from a high-pressure air source to the flexible hose 262. The high-pressure air source may be a high-pressure air tank storing compressed air or an air compressor that compresses air and supplies high-pressure air. The dispenser control device 270 may control the three-way solenoid valve 252, an air compressor 260, and the flow rate control valve. The dispenser control device 270 may control the operation of the three-way solenoid valve 252 to select a nozzle through which the ultrasound gel 30 is to be discharged, from among a pair of nozzles 240 and 242. The dispenser control device 270 may control the flow rate control valve to control the flow rate of the high-pressure air supplied to the hollow housing 220 and control an amount of the ultrasound gel 30 discharged onto the patient's skin surface through the nozzle 240. The dispenser control device 270 may adjust the amount of the ultrasound gel 30 discharged onto the patient's skin surface according to a line scanning speed of the ultrasonic probe 110.

Referring to FIG. 11, when the dispenser control device 270 opens the flow rate control valve installed in the flexible hose 264, high-pressure air is introduced into the hollow housing 220 of the ultrasound gel dispenser 210 through the inlet 220a. The flexible ultrasound gel container 230 is compressed and deformed by the high-pressure air introduced into the hollow housing 220, and the ultrasound gel 30 accommodated in the gel container 230 is discharged and supplied to the nozzle 240 through the flexible hose 256. Instead of supplying the high-pressure air from the high-pressure air tank to the flexible hose 264, by connecting the air compressor 260 to the flexible hose 256, the high-pressure air compressed by the compressor may be supplied directly to the flexible hose 264. In some embodiments, a syringe pump may be used instead of supplying the high-pressure air using a device for controlling the discharge of the ultrasound gel 30 by pressurizing the gel container 230. The syringe pump advances a plunger to the inside of the hollow housing 220 to pressurize, compress, and deform the gel container 230 so that the ultrasound gel 30 is discharged.

Referring to FIG. 1, the medical imaging system 10 according to the present invention may include the computer 400, and the computer 400 is connected to the multi-axis robotic arm control device 310, the ultrasonic image generation device 120, the dispenser control device 270, and the 3D image capturing device 510 and is configured to transmit or receive data and control signals through a communication device 450.

The computer 400 includes an input device 420, a display 430, a memory 440 in which programs are stored, and a processor 410 for executing the programs stored in the memory 440. The input device 420 includes a keyboard, a mouse 424, etc. Further, the computer 400 includes the communication device 450 for transmitting or receiving data and control signals to or from external devices. The communication device 450 may be a wired communication device or a wireless communication device. A 3D shape data generation program 441, a probe path generation program 442, a robotic arm control program 443, an image acquisition control program 444, a dispenser control program 445, and a camera control program 446 are stored in the memory 440. In order to acquire an ultrasonic image of a patient using the multi-axis robotic arm 300, the programs may be executed sequentially or simultaneously by the processor 410.

The computer 400 receives an image of an ultrasound scan area of the patient from the 3D image capturing device 510 and processes the received image of the ultrasound scan area of the patient to generate 3D shape data of the ultrasound scan area. Further, the computer 400 generates an ultrasound scan path for acquiring an ultrasonic image by moving the ultrasonic probe 110 mounted on the multi-axis robotic arm 300 on the basis of the 3D shape data of the ultrasound scan area. The ultrasound scan path includes a movement path of the ultrasonic probe 110, an ultrasonic image acquisition position, a pose of the ultrasonic probe, a moving speed, etc. Further, the computer 400 controls the multi-axis robotic arm control device 310, the ultrasonic image generation device 120, and the dispenser control device 270 to discharge the ultrasound gel 30 while the ultrasonic probe 110 moves along the ultrasound scan path, and at the same time, receive the ultrasonic image acquired from the ultrasonic image generation device 120. The computer 400 executes the robotic arm control program 443 to control the ultrasonic probe 110 to move translationally and/or rotationally along individual ultrasound scan paths L1, L2, ..., and Ln through the robotic arm control device 310, and at the same time, executes the image acquisition control program 444 to control the operation of the ultrasonic probe 110 so that line scanning data is acquired through the ultrasonic image generation device 120. The ultrasonic image generation device 120 transmits the line scanning data acquired from the ultrasonic probe 110 to the computer device 400.

Hereinafter, a method of scanning an ultrasonic image of an ultrasound scan area of a patient using a system using a robotic arm to perform ultrasound examination of a human body according to the present invention will be described.

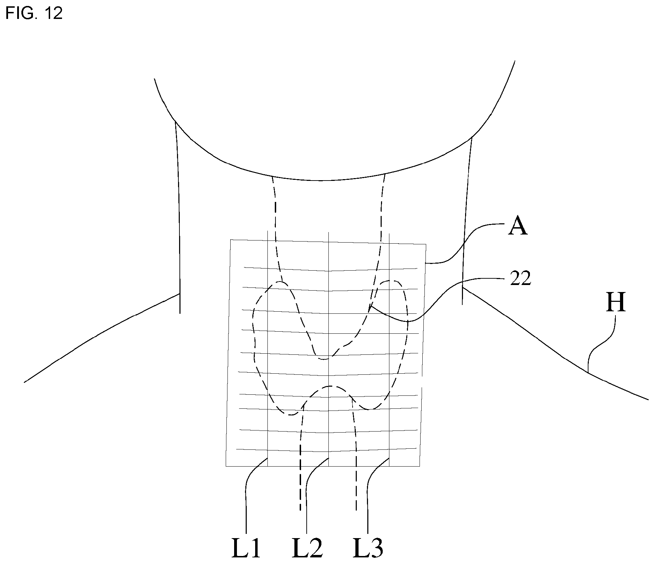

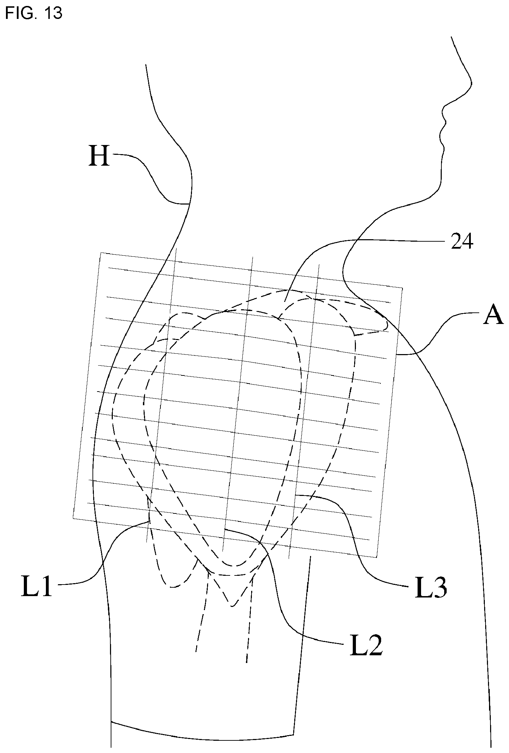

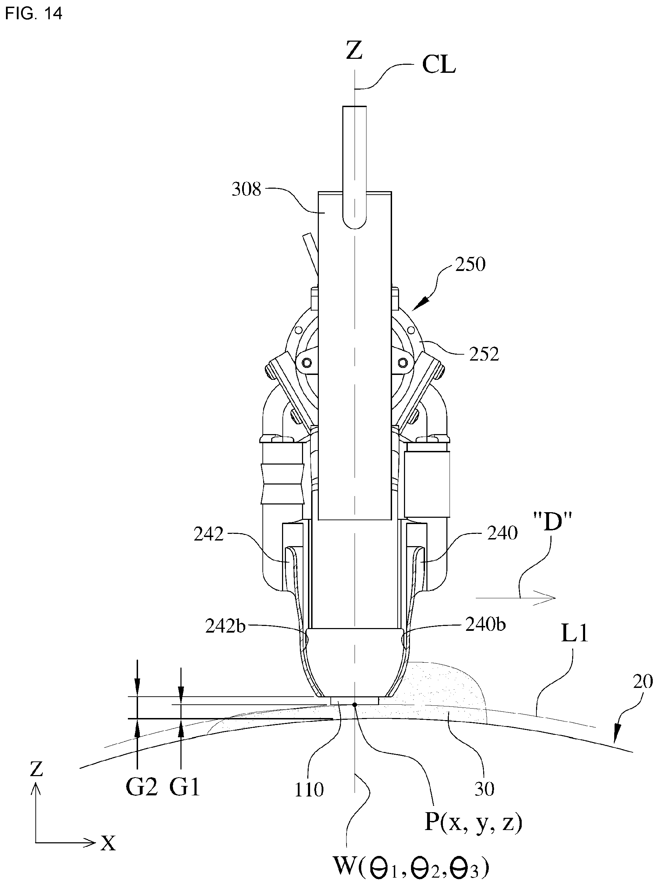

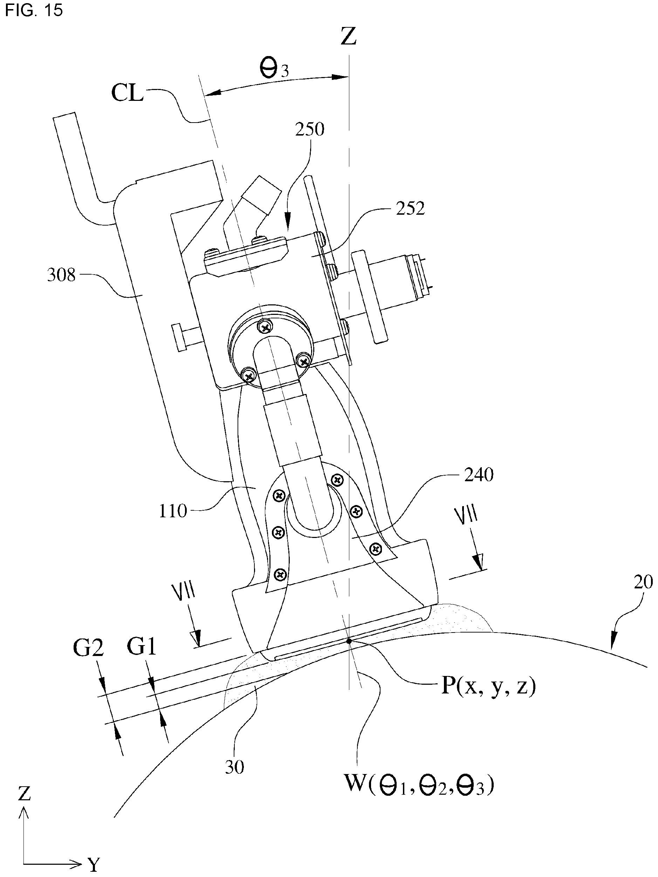

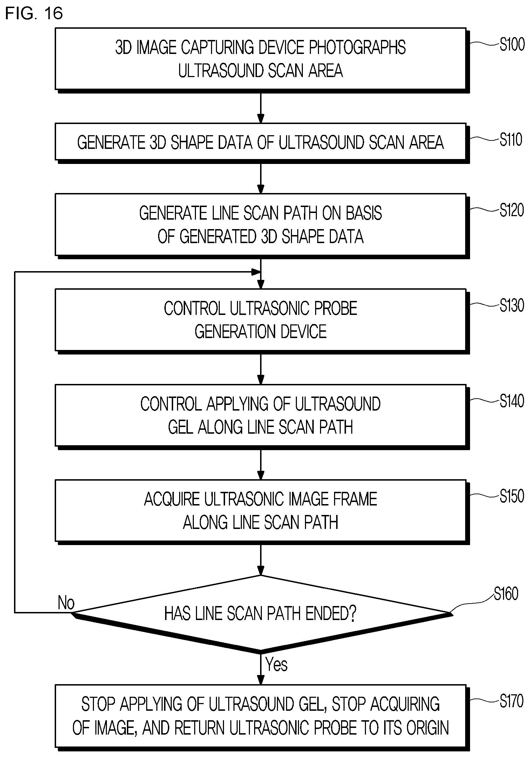

FIG. 12 illustrates an area A for ultrasound scanning of a thyroid gland 22 of a patient H and line scan paths L1, L2, L3, ..., and Ln of an ultrasonic probe. FIG. 13 illustrates an area A for ultrasound scanning of a musculoskeletal system 24 of a shoulder region of a patient H and line scan paths L1, L2, L3, ..., and Ln of an ultrasonic probe. FIGS. 14 and 15 are explanatory diagrams illustrating states in which ultrasound gel is supplied from a nozzle when line scanning is performed on a skin surface with an ultrasonic probe, and FIG. 16 is a flowchart illustrating a multi-axis robot-arm assisted ultrasound medical image acquisition method according to the present invention.

Referring to FIGS. 12 and 13, a skin surface of an ultrasound scan area A of the patient H has a 3D curved shape. In the present embodiment, the multi-axis robotic arm 300 equipped with the ultrasonic probe 110 is a six-axis robotic arm capable of performing ultrasound scanning along the 3D curved shape.

Referring to FIG. 16, when a user of the medical imaging system 10 wants to photograph an ultrasound scan area of a patient, for example, the thyroid gland area of FIG. 12 or the musculoskeletal system area of FIG. 13, the user first photographs the ultrasound scan area A using the 3D image capturing device 510 (S100). The user of the medical imaging system may acquire an image of the ultrasound scan area A while viewing the display of the computer 400. Further, when a 3D camera is mounted on the multi-axis robotic arm 300, the user of the medical imaging system may acquire an image of the ultrasound scan area A by moving the multi-axis robotic arm 300 to a desired position.

Next, the computer 400 receives the image captured by the 3D image capturing device 510 and generates 3D shape data of the ultrasound scan area A with respect to the origin of the robotic arm 300. The 3D shape data generation program 441 is executed in the processor 410, and the 3D shape data of the ultrasound scan area A is generated based on the origin of the robotic arm 300. The generated 3D shape data of the ultrasound scan area A is obtained by representing the skin surface in the ultrasound scan area A in a mesh shape and may be composed of coordinates (x, y, z) of mesh points on the basis of the origin of the robotic arm 300.

Next, the processor 410 of the computer 400 executes the probe path generation program 442 to generate line scan paths L1, L2, and L3 of the ultrasonic probe 110 for the scan area A, as illustrated in FIGS. 12 and 13. The line scan paths L1, L2, and L3 include a movement path of the ultrasonic probe 110, an ultrasonic image acquisition position, a pose of the ultrasonic probe 110, a moving speed, etc.

Next, the processor 410 of the computer 400 simultaneously executes the robotic arm control program 443, the image acquisition control program 444, and the dispenser control program 445 to move the robotic arm 300 equipped with the ultrasonic probe 110 along the line scan paths L1, L2, and L3 and simultaneously supply the ultrasound gel 30 to the skin surface of the patient H and acquire an ultrasonic image (S130, S140, and S150).

In some embodiments, the ultrasonic probe 110 may acquire line scanning data at about 30 frames per second (fps). The line scanning data may include image data and position and pose information of the ultrasonic probe. Further, the line scanning data may include position information of the ultrasonic probe in the 3D shape data. The computer 400 may generate a plurality of picture frames including image data of the line scanning data and the position and pose information of the ultrasonic probe using the image acquisition control program 444.

FIGS. 14 and 15 illustrate states in which ultrasound scanning is performed on the thyroid gland area of the patient H along the scan path L1. Referring to FIG. 14, the scan path L1 is generated such that an end portion of the ultrasonic probe 110 is spaced a certain distance G1 from the patient's skin surface 20. In some embodiments, the coordinates of the scan paths L1, L2, and L3 may be generated by forming a virtual scan path with the 3D surface coordinates of the ultrasound scan area A and adding the predetermined value G1 to the Z-direction coordinates. When the ultrasonic probe 110 moves along the scan path L1 from the nozzle 240 installed forward in a movement direction of the ultrasonic probe 110, the ultrasound gel 30 is continuously supplied to the skin surface 20. Further, the nozzle 240 is installed so that an end portion of the nozzle 240 is positioned further from the patient's skin surface 20 than the end portion of the ultrasonic probe 110. Therefore, when the ultrasound gel 30 is discharged, as illustrated, the ultrasound gel 30 accumulates in front of the ultrasonic probe 110, and when the ultrasound gel 30 is sufficiently supplied, the ultrasound gel 30 that is not brought into contact with air enters a gap G1 between the skin surface 20 and the ultrasonic probe 110 and thus a high-quality ultrasonic image may be acquired. In particular, it is recommended that the ultrasound gel 30 do not contain air. When or after the air-free ultrasound gel 30 is discharged from the nozzle 240, air may be mixed in the air-free ultrasound gel 30 and supplied to a space between the ultrasonic probe and the skin surface. In order to prevent this, a structure in which the nozzle is placed close to the ultrasonic probe and allows new gels to be discharged into the previously discharged gel is required. First, the new gels are discharged into the discharged gel, thereby generating a gel balloon made of ultrasound gel, and achieving the same effect as the end portion of the ultrasonic probe moving in the ultrasound gel balloon. That is, it is possible to achieve the same effect as performing an ultrasound examination underwater. In order to ensure that the ultrasound gel continuously discharged from the nozzle is discharged into the previously discharged ultrasound gel, it is recommended that an end portion of the nozzle be placed as close as possible to an end portion of the ultrasonic probe, and that an outlet of the nozzle be made as thin as possible. It is preferable to make the nozzle have a length greater than the length of the ultrasonic probe, but it does not need to be too long or too short. For example, it is preferable that the length of the outlet of the nozzle be more than or equal to 2/3 and less than or equal to 3.5/3 of the length of the ultrasonic probe. In particular, pressure according to the Reynolds equation is generated on a film formed by the ultrasound gel 30 between the end portion of the ultrasonic probe 110 and the skin surface as the ultrasonic probe 110 moves, which has an effect of slightly pressurizing the skin surface, and thus an effect similar to directly pressing the skin surface with the ultrasonic probe 110 may be obtained.

Referring to FIG. 15, a passage of the nozzle 240 is formed in an inverted funnel shape so that the ultrasound gel 30 spreads wider than the length of the ultrasonic probe 110 and is discharged onto the patient's skin surface 20. As illustrated in FIG. 15, the scan path L1 includes information indicating that the ultrasonic probe 110 is inclined at a certain angle $\theta_3$ with respect to the Z-axis.

Next, whether scanning along the generated scan paths L1, L2, and L3 is completed is determined (S160), and when the ultrasound scanning along all the generated paths is not completed (N in S160), operations S130, S140, and S150 are repeated. When the ultrasound scanning along all the generated paths is completed (Y in S160), the processor 410 of the computer 400 stops applying the ultrasound gel, stops acquiring the ultrasonic image, and returns the ultrasonic probe to its origin (S170).

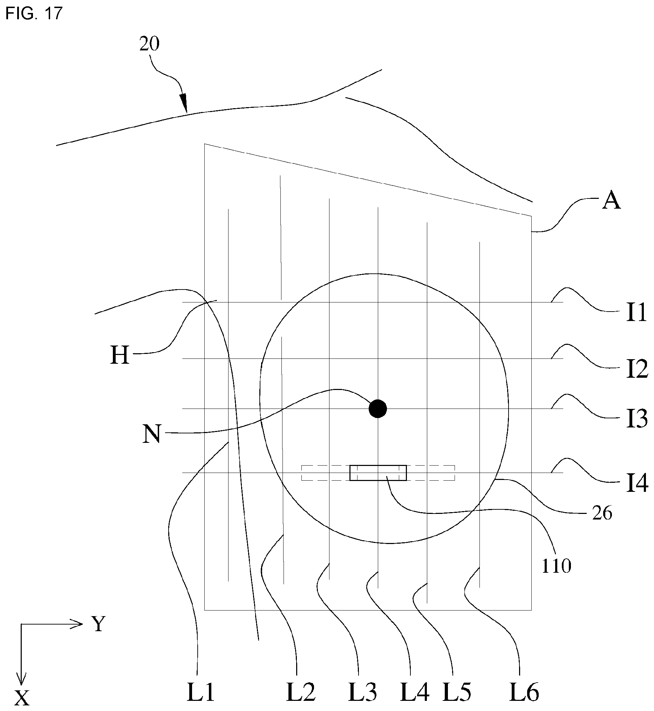

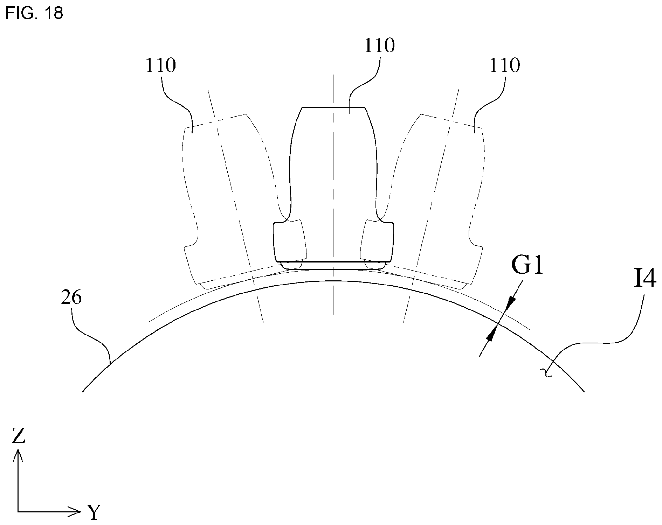

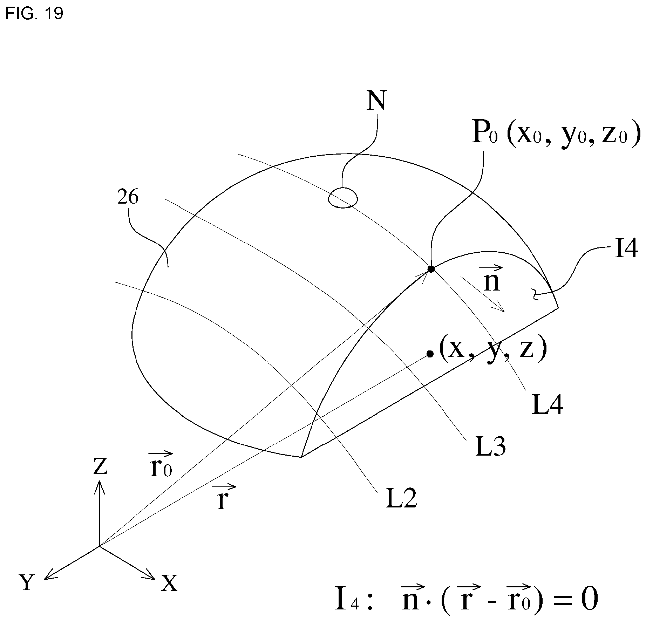

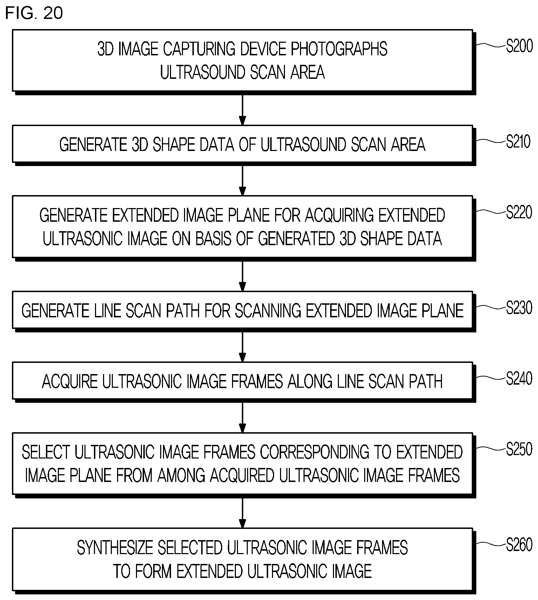

FIG. 17 is an explanatory diagram of a path along which ultrasound scanning is performed on an ultrasound area of a patient's right breast, FIG. 18 is an explanatory diagram illustrating a state in which an ultrasound area of a patient's right breast is scanned in an overlapping manner, and FIG. 19 is an explanatory diagram illustrating an extended image plane. Further, FIG. 20 is a flowchart illustrating a method of forming an extended ultrasonic image according to the present invention.

Referring to FIG. 17, a skin surface of an area of a breast 26 of the patient H undergoing ultrasound scanning has a 3D curved shape. Further a linear array ultrasonic probe has a length of about 4 to 5 cm and cannot acquire an ultrasound image of the entire breast area in a single ultrasound scan. Even when the length of the ultrasonic probe is sufficiently long, it is difficult to scan the ultrasonic image of the entire breast area with a single ultrasound scan because there are areas where the ultrasound gel is not completely applied due to the skin surface that is to be scanned and has a 3D curved shape.

Providing the ultrasonic image of the entire cross section of the breast area of the patient may be useful in diagnosing the patient's disease. In order to provide the ultrasonic image of the entire cross section of the breast area, a plurality of cross-sectional images with respect to the same surface may be synthesized and provided. Using a multi-axis robotic arm, a plurality of cross-sectional ultrasound images may be acquired at a desired position, which can be advantageous for extended ultrasonic image synthesis.

Hereinafter, a method of scanning a skin surface with a 3D curved shape to provide extended ultrasonic images is provided. The extended ultrasonic images are cross-sectional images on the same surface, capable of viewing the entire thyroid gland, musculoskeletal system, and breast areas, and are ultrasonic images formed by synthesizing a plurality of image frames.

FIG. 17 illustrates a scan area A for ultrasound scanning of a right breast 26 of the patient H and line scan paths L1, L2, L3, L4, L5, and L6 indicated in the scan area A. The line scan paths L1, L2, L3, L4, L5, and L6 are paths for acquiring an ultrasonic image of the breast area of the patient by moving the ultrasonic probe 110 mounted on the robotic arm 300. As illustrated in FIG. 17, intervals between the paths are shorter than the length of the ultrasonic probe 110, that is, are formed so that ultrasonic image frames acquired when scanning along the scan paths with the ultrasonic probe 110 are photographed to overlap. Further, FIG. 17 illustrates extended image planes I1, I2, I3, and I4 to be acquired by synthesizing the ultrasonic images acquired from the line scan paths L1, L2, L3, L4, L5, and L6.

FIGS. 18 and 19 are explanatory diagrams illustrating the extended image plane I4. As illustrated in FIG. 18, ultrasound scan paths are formed to have intervals therebetween so that the ultrasonic probe 110 overlaps. Further, when the ultrasound gel supply device is mounted on the robotic arm, as illustrated in FIG. 18, the ultrasound scan paths may be generated so that the ultrasonic probe 110 is spaced a certain distance G1 from a skin surface 26. In the case of a robotic arm that does not have an ultrasound gel supply device, a user may apply ultrasound gel on a skin surface in advance, and ultrasound scan paths may be generated so that the ultrasound scan paths generated by a computer are brought into close contact with the skin surface.

Referring to FIG. 19, the extended image plane I4 may be expressed in the following equation.

$$[\text{Equation 1}]$$

$$I_4 : \ \vec{n} \cdot (\vec{r} - \vec{r_0}) = 0$$

Here, $r_0$ denotes a distance vector from the origin of reference coordinates of the robotic arm 300 to a point $P_0$ ($X_0$, $Y_0$, $Z_0$) on the scan path L4, and r denotes a distance vector of an arbitrary position on the extended image plane I4. Further, n denotes a normal vector of the extended image plane I4. As illustrated in FIGS. 18 and 19, in the present embodiment, the extended image plane I4 is a plane parallel to a Z-Y plane.

Hereinafter, a method of acquiring an extended ultrasonic image will be described with reference to FIGS. 1 to 3 and 20.

The extended ultrasonic image may be acquired using a multi-axis robotic arm system according to the present invention having an automatic ultrasound gel supply device, as illustrated in FIGS. 1 to 3. Further, the extended ultrasonic image may be acquired using a multi-axis robotic arm system that does not have an automatic ultrasound gel supply device. When the automatic ultrasound gel supply device is not provided, a user should apply ultrasound gel on a patient's skin surface in advance.

First, an ultrasound scan area A of the patient is photographed by a 3D image capturing device 510 so that a 3D image of the ultrasound scan area A is acquired (S200).

Next, a computer 400 receives an image of the ultrasound scan area from the 3D image capturing device 510 and executes a 3D shape data generation program 441 to generate 3D shape data of the ultrasound scan area (S210).

Next, extended image planes I1, I2, I3, and I4 are generated to acquire the extended ultrasonic image from the 3D shape data generated by the computer 400 (S220). The extended ultrasonic image has a preferred interval and angle according to the organ on which ultrasonic image capturing is performed. For example, in the case of acquiring extended ultrasonic images of a breast, a thyroid gland, etc., it is preferable to generate extended image planes parallel to each other that are perpendicular to an extending direction of a center axis of an ultrasonic probe. Since ultrasonic images have good quality when the ultrasonic probe is perpendicular to the skin surface, when a scan path of the ultrasonic probe is curved, it is preferable to form extended image planes so that the extended image planes form predetermined angles while being perpendicular to the ultrasonic probe's movement path. The extended image plane I4 illustrated in FIG. 19 is generated at a certain interval to be parallel to the Z-Y plane of a reference coordinate system of a robotic arm 300.

Next, the computer 400 executes a probe path generation program 442 to generate ultrasound scan paths L1, L2, L3, L4, L5, and L6 for acquiring a plurality of ultrasonic image frames including overlapping areas for the extended image planes (S230). When the automatic ultrasound gel supply device is mounted on the multi-axis robotic arm system 300, the ultrasound scan paths L1, L2, L3, L4, L5, and L6 are generated so that the ultrasonic probe 110 is spaced a certain distance from the skin surface.

Next, the computer 400 controls the robotic arm 300 equipped with the ultrasonic probe 110 to move the ultrasonic probe 110 along the generated line scan paths L1, L2, L3, L4, L5, and L6 and acquire a plurality of ultrasonic image frames (S240).

The computer 400 may execute an image acquisition control program 444 to generate a plurality of picture frames F1, F2, ..., and Fn including image data of line scanning data and position and pose information of the ultrasonic probe. Each of the acquired ultrasonic image frames includes information on scan path L1 to L6 of the ultrasonic probe 110 when the ultrasonic image is acquired, extended image plane information I1 to I4, position information, pose information of the ultrasonic probe, etc. The position and pose information of the ultrasonic probe may be expressed as coordinates of a coordinate system based on a multi-axis robotic arm. For example, coordinates P (X, Y, X) of a center point of an end portion of the ultrasonic probe 110 of FIG. 15 and an inclination angle W ($\theta_1$, $\theta_2$, $\theta_3$) of a center axis CL of the ultrasonic probe 110 with respect to the X, Y, and Z axes, respectively, may be used.

When a plurality of ultrasonic image frames are acquired using the multi-axis robotic arm system 10 equipped with the automatic ultrasound gel supply device, the computer 400 may acquire ultrasonic image frames while simultaneously controlling the multi-axis robotic arm, the ultrasonic probe mounted on the multi-axis robotic arm, and the ultrasound gel supply device. In this case, while the ultrasonic probe 110 moves along the ultrasound scan path, as illustrated in FIGS. 14 and 15, the computer 400 discharges the ultrasound gel 30 in front of a movement path of the ultrasonic probe 110 and simultaneously acquires an ultrasonic image. In this case, the ultrasound scan path generated by the computer 400 is formed so that the end portion of the ultrasonic probe 110 is spaced a certain distance G1 from a skin surface of the ultrasound scan area.

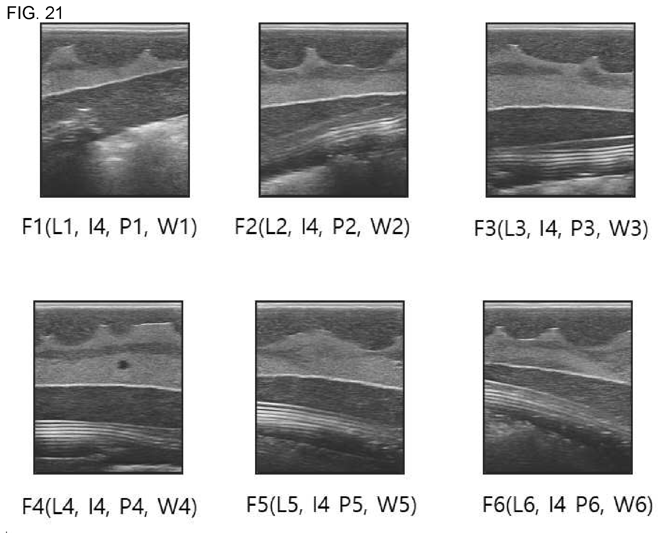

Next, ultrasonic image frames corresponding to each of the extended image planes I1 to I4 are selected from among the plurality of ultrasonic image frames acquired by the computer 400 (S250). FIG. 21 illustrates ultrasonic image frames for each selected image path corresponding to the extended image plane I4. Each ultrasonic image frame includes path information L1 to L6, extended image plane information I4, and position P and pose W information of the ultrasonic probe when each ultrasonic image is captured.

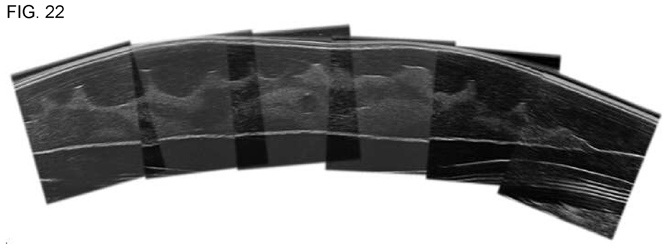

Next, the computer 400 synthesizes overlapping portions of the plurality of selected ultrasonic image frames to generate an extended ultrasonic image (S260). FIG. 22 illustrates the extended ultrasonic image generated by synthesizing the respective ultrasonic image frames illustrated in FIG. 21. The extended ultrasonic image may be synthesized using the position and pose information of the ultrasonic probe and overlapping information of paths that are included in each ultrasonic image frame. Alternatively, the extended ultrasonic image may be synthesized using machine learning or numerical computation algorithms. The computer 400 may execute an image processing program to generate an extended ultrasonic image (or panoramic images) from picture frames. Further, the computer 400 may remove the overlapping portions between the image frames (e.g., L1 and L2) photographed on adjacent paths by a stitching algorithm in order to generate panoramic images by executing the image processing program.

The above-described embodiments are not intended to limit the scope of the present invention. Those skilled in the art may modify, alter, or substitute various embodiments within the scope of the claims, beyond those described herein, and it should be understood that such modifications fall within the scope of the present invention.

**Claims**

1.  A robot-arm assisted ultrasound medical imaging system comprising:

    a multi-axis robotic arm;
    a multi-axis robotic arm control device that controls the multi-axis robotic arm;
    an ultrasonic probe mounted on the multi-axis robotic arm;
    an ultrasonic image generation device that controls the ultrasonic probe to generate an ultrasonic image;
    a three-dimensional (3D) image capturing device;
    an ultrasound gel dispenser that is mounted on the multi-axis robotic arm and in which ultrasound gel is accommodated;
    a nozzle disposed adjacent to the ultrasonic probe to receive the ultrasound gel from the ultrasound gel dispenser and discharge the received ultrasound gel forward in a movement direction of the ultrasonic probe;

a dispenser control device that controls discharge of the ultrasound gel accommodated in the ultrasound gel dispenser; and

a computer that controls the multi-axis robotic arm control device, the ultrasonic image generation device, the 3D image capturing device, and the dispenser control device,

wherein the computer is configured to receive an image of an ultrasound scan area of a patient from the 3D image capturing device,

process the image of the ultrasound scan area of the patient to generate 3D shape data of the ultrasound scan area,

generate an ultrasound scan path for acquiring the ultrasonic image by moving the ultrasonic probe mounted on the multi-axis robotic arm on the basis of the 3D shape data of the ultrasound scan area, and

control the multi-axis robotic arm control device, the ultrasonic image generation device, and the dispenser control device to discharge the ultrasound gel and simultaneously acquire the ultrasonic image while the ultrasonic probe moves along the ultrasound scan path.

2. The robot-arm assisted ultrasound medical imaging system of claim 1, wherein the ultrasound scan path generated by the computer is formed such that an end portion of the ultrasonic probe is spaced a certain distance from a surface of an ultrasound scan area of the patient.

3. The robot-arm assisted ultrasound medical imaging system of claim 1, wherein the ultrasound gel dispenser includes:

a hollow housing having an inlet formed at one side thereof and connected to an air source to allow high-pressure air to be introduced and an opening formed at the other side to allow a flexible ultrasound gel container to be inserted therein, and mounted on the multi-axis robotic arm; and

a hollow housing cap having a small diameter portion formed to allow an outlet of the flexible ultrasound gel container inserted therein to be inserted and a large diameter portion formed to be sealed and coupled to the opening of the hollow housing,

wherein the dispenser control device includes a valve for allowing or blocking a flow of air of the air source supplied to the hollow housing of the ultrasound gel dispenser.

4. The robot-arm assisted ultrasound medical imaging system of any one claim of claims 1 to 3, wherein the ultrasonic probe is a linear array probe, and

the nozzle includes an inlet, an outlet, and a passage that connects the inlet and the outlet, wherein the passage is formed such that a length of a cross section thereof increases from the inlet to the outlet.

5. The robot-arm assisted ultrasound medical imaging system of claim 4, wherein an end portion of the nozzle is disposed to be spaced a certain distance from the end portion of the ultrasonic probe so as to be positioned further from the patient's skin surface than the end portion of the ultrasonic probe.

6. The robot-arm assisted ultrasound medical imaging system of claim 4, wherein the nozzle is formed to surround a side surface in a width direction of the linear array probe so that the side surface in the width direction of the linear array probe defines the passage.

7. A robot-arm assisted ultrasound medical image acquisition method which is an ultrasound medical image acquisition method using a multi-axis robotic arm, the robot-arm assisted ultrasound medical image acquisition method comprising:

receiving, by a computer, an image of an ultrasound scan area of a patient from a depth imaging device;

processing, by the computer, the received image of the ultrasound scan area of the patient and generating 3D shape data of the ultrasound scan area;

generating, by the computer, an ultrasound scan path for acquiring an ultrasonic image by moving an ultrasonic probe mounted on a multi-axis robotic arm on the basis of the generated 3D shape data of the ultrasound scan area; and

controlling, by the computer, the multi-axis robotic arm, the ultrasonic probe mounted on the multi-axis robotic arm, and an ultrasound gel supply device, and discharging ultrasound gel forward in a movement direction of the ultrasonic probe and simultaneously acquiring the ultrasonic image while the ultrasonic probe moves along the ultrasound scan path.

8. The robot-arm assisted ultrasound medical image acquisition method of claim 7, wherein the ultrasound scan path generated by the computer is formed such that an end portion of the ultrasonic probe is spaced a certain distance (G1) from a surface of the ultrasound scan area of the patient.

9. The robot-arm assisted ultrasound medical image acquisition method of claim 7 or 8, wherein the ultrasound gel supply device includes an ultrasound gel dispenser that is mounted on the multi-axis robotic arm and in which ultrasound gel is accommodated, a nozzle disposed adjacent to the ultrasonic probe to receive the ultrasound gel from the ultrasound gel dispenser and discharge the received ultrasound gel forward in the movement direction of the ultrasonic probe, and a dispenser control device that controls discharge of the ultrasound gel accommodated in the ultrasound gel dispenser, and
the computer is configured to control the dispenser control device to discharge the ultrasound gel.

10. The robot-arm assisted ultrasound medical image acquisition method of claim 7 or 8, wherein the ultrasonic probe is a linear array probe, and
the computer is configured to control the ultrasound gel dispenser to discharge the ultrasound gel so that a certain distance (G1) between an end portion of the linear array probe and the surface of the ultrasound scan area of the patient is greater than a product of a length of the linear array probe and a moving speed of the linear array probe.

FIG. 1

FIG. 2

FIG. 3

FIG. 4

FIG. 5

FIG. 6

**EP 4 781 915 A1**

FIG. 7

20

FIG. 8

FIG. 9

FIG. 10

FIG. 11

COMPRESSED AIR

FIG. 12

FIG. 13

FIG. 14

FIG. 15

FIG. 16

```
┌──────────────────────────────────────────┐
│   3D IMAGE CAPTURING DEVICE PHOTOGRAPHS   │──S100
│          ULTRASOUND SCAN AREA             │
└──────────────────────────────────────────┘
                    │
                    ▼
┌──────────────────────────────────────────┐
│  GENERATE 3D SHAPE DATA OF ULTRASOUND     │──S110
│              SCAN AREA                     │
└──────────────────────────────────────────┘
                    │
                    ▼
┌──────────────────────────────────────────┐
│    GENERATE LINE SCAN PATH ON BASIS       │──S120
│       OF GENERATED 3D SHAPE DATA          │
└──────────────────────────────────────────┘
                    │
                    ▼
┌──────────────────────────────────────────┐
│        CONTROL ULTRASONIC PROBE           │──S130
│           GENERATION DEVICE               │
└──────────────────────────────────────────┘
                    │
                    ▼
┌──────────────────────────────────────────┐
│     CONTROL APPLYING OF ULTRASOUND        │──S140
│        GEL ALONG LINE SCAN PATH           │
└──────────────────────────────────────────┘
                    │
                    ▼
┌──────────────────────────────────────────┐
│      ACQUIRE ULTRASONIC IMAGE FRAME       │──S150
│          ALONG LINE SCAN PATH             │
└──────────────────────────────────────────┘
                    │
                    ▼
        No  ◇─────────────────────◇  S160
    ┌────────  HAS LINE SCAN PATH ENDED?  ────
    │       ◇─────────────────────◇
    │                │
    │               Yes
    │                ▼
    │  ┌──────────────────────────────────────────┐
    │  │ STOP APPLYING OF ULTRASOUND GEL, STOP     │──S170
    │  │ ACQUIRING OF IMAGE, AND RETURN            │
    │  │ ULTRASONIC PROBE TO ITS ORIGIN            │
    │  └──────────────────────────────────────────┘
```

FIG. 17

FIG. 18

FIG. 19

$$I_4: \quad \vec{n} \cdot (\vec{r} - \vec{r_0}) = 0$$

FIG. 20

| 3D IMAGE CAPTURING DEVICE PHOTOGRAPHS ULTRASOUND SCAN AREA | S200 |

↓

| GENERATE 3D SHAPE DATA OF ULTRASOUND SCAN AREA | S210 |

↓

| GENERATE EXTENDED IMAGE PLANE FOR ACQUIRING EXTENDED ULTRASONIC IMAGE ON BASIS OF GENERATED 3D SHAPE DATA | S220 |

↓

| GENERATE LINE SCAN PATH FOR SCANNING EXTENDED IMAGE PLANE | S230 |

↓

| ACQUIRE ULTRASONIC IMAGE FRAMES ALONG LINE SCAN PATH | S240 |

↓

| SELECT ULTRASONIC IMAGE FRAMES CORRESPONDING TO EXTENDED IMAGE PLANE FROM AMONG ACQUIRED ULTRASONIC IMAGE FRAMES | S250 |

↓

| SYNTHESIZE SELECTED ULTRASONIC IMAGE FRAMES TO FORM EXTENDED ULTRASONIC IMAGE | S260 |

FIG. 21

F1(L1, I4, P1, W1)  F2(L2, I4, P2, W2)  F3(L3, I4, P3, W3)

F4(L4, I4, P4, W4)  F5(L5, I4 P5, W5)  F6(L6, I4 P6, W6)

FIG. 22

## INTERNATIONAL SEARCH REPORT

| International application No. |
| --- |
| **PCT/KR2025/014323** |

### A. CLASSIFICATION OF SUBJECT MATTER

**A61B 8/00**(2006.01)i

According to International Patent Classification (IPC) or to both national classification and IPC

### B. FIELDS SEARCHED

Minimum documentation searched (classification system followed by classification symbols)

A61B 8/00(2006.01); A61B 50/00(2016.01); A61M 35/00(2006.01); B67D 7/06(2010.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (MOIP internal) & keywords: 초음파 의료 영상화(medical ultrasonic imaging), 다축 로봇 아암(multi-axes robotic arm), 3차원 이미지(three-dimensional image), 스캔 경로(scan path), 초음파 젤 디스펜서(ultrasound gel dispenser), 노즐(nozzle), 이격된 프로브(spaced probe)

### C. DOCUMENTS CONSIDERED TO BE RELEVANT

| Category* | Citation of document, with indication, where appropriate, of the relevant passages | Relevant to claim No. |
| --- | --- | --- |
| X | JP 2023-160379 A (SUMITOMO HEAVY INDUSTRIES LTD.) 02 November 2023 (2023-11-02)<br>see abstract; paragraphs [0009], [0028]-[0076]; and figures 1-2, 10, 12. | 1,7,9 |
| Y | | 3 |
| A | | 2,4-6,8,10 |
| Y | US 2010-0155431 A1 (BARTOLUCCI, STEFANO et al.) 24 June 2010 (2010-06-24)<br>see abstract; paragraphs [0001], [0016], [0024], [0054]-[0055]; claim 15; and figures 16A-16B. | 3 |
| A | KR 10-2008-0058774 A (MEDISON CO., LTD.) 26 June 2008 (2008-06-26)<br>see entire document. | 1-10 |
| A | KR 10-2023-0089656 A (HAN, Min Soo) 21 June 2023 (2023-06-21)<br>see entire document. | 1-10 |

☑ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **12 December 2025** | **14 December 2025** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
| --- |
| **PCT/KR2025/014323** |

| C. | DOCUMENTS CONSIDERED TO BE RELEVANT | |
| --- | --- | --- |
| Category* | Citation of document, with indication, where appropriate, of the relevant passages | Relevant to claim No. |
| A | CN 116919455 A (ZHEJIANG NORMAL UNIVERSITY) 24 October 2023 (2023-10-24)<br>see entire document. | 1-10 |
| PY | KR 10-2025-0062002 A (MEDICALPARK CO., LTD.) 08 May 2025 (2025-05-08)<br>see abstract; paragraphs [0016], [0037]-[0052]; claim 4; and figures 6-10. | 3 |

Form PCT/ISA/210 (second sheet) (July 2022)

**INTERNATIONAL SEARCH REPORT**
**Information on patent family members**

International application No.

**PCT/KR2025/014323**

| Patent document cited in search report | | | Publication date (day/month/year) | Patent family member(s) | | | Publication date (day/month/year) |
|---|---|---|---|---|---|---|---|
| JP | 2023-160379 | A | 02 November 2023 | None | | | |
| US | 2010-0155431 | A1 | 24 June 2010 | EP | 2202179 | A1 | 30 June 2010 |
| | | | | EP | 2202179 | B1 | 20 June 2012 |
| | | | | ES | 2389744 | T3 | 31 October 2012 |
| | | | | US | 8511924 | B2 | 20 August 2013 |
| | | | | WO | 2010-075119 | A1 | 01 July 2010 |
| KR | 10-2008-0058774 | A | 26 June 2008 | KR | 10-0875621 | B1 | 26 December 2008 |
| KR | 10-2023-0089656 | A | 21 June 2023 | None | | | |
| CN | 116919455 | A | 24 October 2023 | None | | | |
| KR | 10-2025-0062002 | A | 08 May 2025 | WO | 2025-095512 | A1 | 08 May 2025 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20120271173 A1

- US 20080021317 A1